# EUROPEAN PATENT APPLICATION

(11) **EP 4 603 087 A1**
(43) Date of publication of application: **20.08.2025**
(21) Application number: 23894415.1
(22) Date of filing: 07.11.2023
(51) Int. Cl.: A61K 31/16, A61K 31/385, A61K 38/08, A61K 47/40, A61P 13/12

(54) **PROPHYLACTIC OR THERAPEUTIC AGENT FOR ACUTE KIDNEY INJURY INDUCED BY ANTICANCER AGENT**

(30) Priority: 21.11.2022 JP 2022185887
(71) Applicant: Kyowa Hakko Bio Co., Ltd., Tokyo 100-8185 (JP); Tokushima University, Tokushima-shi, Tokushima 770-8501 (JP)
(72) Inventor: OHSHIMA Etsuo, Tokyo 100-8185 (JP); ISHIMA Yu, Tokushima-shi, Tokushima 770-8501 (JP)
(74) Representative: Vossius & Partner Patentanwälte Rechtsanwälte mbB
(86) International application number: PCT/JP2023/040110
(87) International publication number: WO 2024/111404

(57) **Abstract**

Disclosed is a prophylactic or therapeutic agent that is for acute kidney injury induced by an anticancer agent and that contains at least one selected from the group consisting of: glutathione trisulfide and pharmacologically acceptable salts thereof; at least one cyclodextrin inclusion complex selected from the group consisting of lipoic acid trisulfide, derivatives of the same, and pharmacologically acceptable salts thereof; lipoic acid trisulfide and derivatives thereof; and pantethine trisulfide and pharmacologically acceptable salts thereof. The present invention also discloses a method for determining, on the basis of a blood GDF-15 level, at least one of the dosage, the dosage regimen, and the administration feasibility of the prophylactic or therapeutic agent.

## Description

### Technical Field

The present invention relates to a prophylactic or therapeutic agent for acute kidney injury induced by an anti-cancer agent.

### Background Art

Acute kidney injury is feared because it significantly impairs patient QOL and exhibits a very high mortality rate. Acute kidney injury is considered to be a state in which severe renal dysfunction occurs as a result of diverse causes operating in combination. Although these causes can be generally referred to as various stresses imposed on the body, in recent years, growth differentiation factor-15 (GDF-15) has attracted attention as a stress marker that responds sensitively to a state in which such various stresses are imposed on the body (Non-Patent Literature 1). It has also been reported that, in ischemic renal injury, GDF-15 rapidly increases after ischemia-reperfusion (Non-Patent Literature 2). Further, it has been reported that the blood GDF-15 level is a predictive marker for poor prognosis in renal injury (Non-Patent Literature 3).

One known cause of acute kidney injury is an anti-cancer agent. Among anti-cancer agents, some directly damage tubular cells and induce acute tubular necrosis (ATN). Examples of drugs that induce ATN include cisplatin, ifosfamide, azacitidine, and imatinib. For example, cisplatin is one of the most frequently used platinum-based anti-cancer drugs for cancer treatment. However, there is a substantial clinical problem in that approximately 20% to 30% of patients receiving cisplatin develop acute kidney injury due to ATN.

It is thought that cisplatin causes necrosis of proximal tubular by active uptake of the drug into cells via organic cation transporters (OCTs) located on the basolateral membrane of proximal tubules. Because kidney injury caused by an anti-cancer agent such as cisplatin is best prevented, guidelines recommend thorough hydration, primarily with physiological saline solution (the guidelines stipulate at least three liters per day) as a prophylaxis. Although the use of diuretics, which have long been widely employed, with established safety, may also be considered, from the perspective of preventing kidney injury, the guidelines only "weakly recommend (suggest)" such an approach (Non-Patent Literature 4).

With respect to side effects such as renal dysfunction caused by administration of cisplatin, there is a report that certain glutathione monoalkyl esters are effective (Patent Literature 1). However, Patent Literature 1 merely describes that, when glutathione monoisopropyl ester sulfate is administered intraperitoneally at a dose of 300 mg/kg, it alleviates cisplatin-induced body weight suppression and elevated blood urea nitrogen. Administering a large quantity of drug by a parenteral route, as in Patent Literature 1, imposes a heavy burden on patients, and thus is not recommended in current guidelines.

There is also a report that Na₂S₄, an inorganic polysulfur compound, is effective for cisplatin-induced renal injury (Non-Patent Literature 5). Although the administration dose of Na₂S₄ is as low as 5.6 mg/kg, the route of administration is intraperitoneal, which can be expected to heavily burden the patient.

Meanwhile, attention has been drawn to favorable effects of organic polysulfur compounds on the human body. It has been reported that organic polysulfur compounds may have physiological functions such as anti-aging effects (for example, Non-Patent Literatures 6 and 7). It has also been reported that some organic polysulfur compounds are involved in mitochondrial functions such as mitochondrial energy production (Non-Patent Literature 8). Examples of organic polysulfur compounds include glutathione trisulfide (hereinafter also referred to as GSSSG) (Patent Literature 2), pantethine trisulfide (Patent Literature 3), and lipoic acid trisulfide (Patent Literature 4). These organic polysulfur compounds can be produced by, for example, the methods described in Patent Literatures 2 and 5. It has also been shown, for example, that GSSSG in combination with other drugs enhances antiinflammatory effects (Patent Literature 6).

### Citation List

### Patent Literature

[Patent Literature 1] JP 63-284132A
[Patent Literature 2] WO 2018/117186
[Patent Literature 3] WO 2022/045052
[Patent Literature 4] WO 2022/045212
[Patent Literature 5] WO 2021/200487
[Patent Literature 6] WO 2022/168975

### Non Patent Literature

[Non-Patent Literature 1] Y. Fujita, et al., Geriatr. Gerontol. Int, 2016, Mar; 16, Suppl, 1: 17-29.
[Non-Patent Literature 2] J. Liu, et al., J. Am. Soc. Nephrol, 2020, Apr; 31(4): 701-715.
[Non-Patent Literature 3] J.-H. Lim, et al., J. Clin Med, 2021, Aug, 18; 10(16): 3660.
[Non-Patent Literature 4] Y. Matsubara, M. Yanagita, "Anti-cancer Drugs and Acute Kidney Injury," The Journal of the Japanese Society of Internal Medicine, 107: 865-871, 2018.
[Non-Patent Literature 5] X. Cao, et al., Redox Biology, Volume 15, May 2018: 513-521.
[Non-Patent Literature 6] T. Ida, et al., PNAS, May 27, 2014, 111(21) 7606-7611.
[Non-Patent Literature 7] T. Akaike, et al., Nature Communications, 2017, Oct 27; 8(1): 1177.
[Non-Patent Literature 8] S. Fujii, et al., Br. J. Pharmacol, 2019, Feb; 176(4): 607-615.

### Summary of Invention

### Technical Problem

An object of the present invention is to provide a prophylactic or therapeutic agent for acute kidney injury induced by an anti-cancer agent, which can exert prophylactic or therapeutic effects at a low dose and has the potential to be administered orally or parenterally.

### Solution to Problem

The present inventors conducted an *in vitro* study of cisplatin-induced cytotoxicity using renal tubular cells, and discovered that an organic trisulfide may have a cell death-suppressing effect about 1000 times stronger than Na₂S₄, an inorganic polysulfide. The present inventors then examined the effect of GSSSG on a cisplatin-induced acute kidney injury model animal. The result suggested that the elevation of blood urea nitrogen (BUN) and serum creatinine (CRE) levels could be suppressed in the administration group. In addition, the present inventors found that measuring the blood GDF-15 level could be useful in selecting at least one of administration propriety, dosage, or administration of: a compound represented by Formula (1) below (hereinafter also referred to as "Compound (1)"); a compound represented by Formula (2) below (hereinafter also referred to as "Compound (2)"); a cyclodextrin clathrate of at least one selected from the group consisting of a pharmacologically acceptable salt of Compound (2), Compound (1), and Compound (2); and pantethine trisulfide and a pharmacologically acceptable salt thereof. 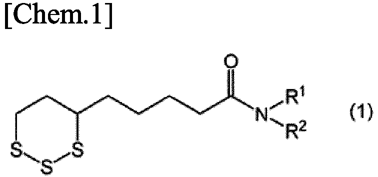 [In the formula, R¹ and R² are each independently a hydrogen atom; a C₁₋₆ alkyl group optionally having one or more substituents selected from the group consisting of a carboxy group and -OR⁵; a C₂₋₆ alkyl group having one or more substituents selected from the group consisting of - NR⁶R⁷ and -N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5] 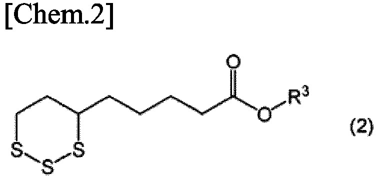 [In the formula, R³ is a hydrogen atom; a C₁₋₆ alkyl group optionally having one or more substituents selected from the group consisting of a carboxy group and -OR⁵; a C₂₋₆ alkyl group having one or more substituents selected from the group consisting of -NR⁶R⁷ and - N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5].

Based on these discoveries, the present inventors have completed the present invention, which includes [1] to [15] below.
[1] A prophylactic or therapeutic agent for acute kidney injury induced by an anti-cancer agent, comprising at least one selected from the group consisting of: GSSSG and a pharmacologically acceptable salt thereof; Compound (1); Compound (2); a cyclodextrin clathrate of at least one selected from the group consisting of a pharmacologically acceptable salt of Compound (2), Compound (1) and Compound (2); and pantethine trisulfide and a pharmacologically acceptable salt thereof (the above being collectively referred to as "trisulfides" in some cases).
[1a] A prophylactic or therapeutic agent for acute kidney injury, comprising the above trisulfides, wherein the agent is administered in combination with an anti-cancer agent.
[1b] An anti-cancer agent, wherein the agent is administered in combination with the above trisulfides.
[2] A method for preventing or treating acute kidney injury induced by an anti-cancer agent, comprising administering the above trisulfides to a patient in need thereof.
[2a] A method for treating cancer, comprising administering the above trisulfides and an anti-cancer agent to a patient in need thereof.
[3] The above trisulfides for use in preventing or treating acute kidney injury induced by an anti-cancer agent.
[3a] The above trisulfides for use in preventing or treating acute kidney injury, wherein the trisulfides are administered in combination with an anti-cancer agent.
[3b] An anti-cancer agent for use in treating cancer, wherein the agent is administered in combination with the above trisulfides.
[3c] A combination of the above trisulfides and an anti-cancer agent for use in treating cancer.
[4] Use of the above trisulfides for the manufacture of a prophylactic or therapeutic agent for acute kidney injury induced by an anti-cancer agent.
[4a] Use of the above trisulfides, for the manufacture of a prophylactic or therapeutic agent for acute kidney injury, wherein the agent is administered in combination with an anti-cancer agent.
[4b] A combination of the above trisulfides and an anti-cancer agent, for treating cancer.
[5] The prophylactic or therapeutic agent, the prophylactic or therapeutic method, the trisulfides for use, or the use of any of [1] to [4b], wherein the above trisulfides are at least one selected from the group consisting of: GSSSG and a pharmacologically acceptable salt thereof; Compound (1); Compound (2); and pantethine trisulfide and a pharmacologically acceptable salt thereof.
[6] The prophylactic or therapeutic agent, the prophylactic or therapeutic method, the trisulfides for use, or the use of any of [1] to [5], wherein the acute kidney injury induced by the anti-cancer agent is an acute kidney injury due to acute tubular necrosis induced by the anti-cancer agent.
[7] The prophylactic or therapeutic agent, the prophylactic or therapeutic method, the trisulfides for use, or the use of any of [1] to [6], wherein the anti-cancer agent is at least one selected from the group consisting of cisplatin, carboplatin, oxaliplatin, cyclophosphamide, ifosfamide, methotrexate, pemetrexed, gemcitabine, pentostatin, and mitomycin C.
[8] The prophylactic or therapeutic agent, the prophylactic or therapeutic method, the trisulfides for use, or the use of any of [1] to [7], wherein the anti-cancer agent is at least one selected from the group consisting of cisplatin, carboplatin, and oxaliplatin.
[9] A method for predicting or determining, for a subject, at least one of administration propriety, dosage, or administration of at least one selected from the group consisting of: Compound (1); Compound (2); a cyclodextrin clathrate of at least one selected from the group consisting of a pharmacologically acceptable salt of Compound (2), Compound (1) and Compound (2); and pantethine trisulfide and a pharmacologically acceptable salt thereof, the method comprising: a measurement step of measuring a blood GDF-15 level of a subject; and a prediction or determination step of predicting or determining, based on the blood GDF-15 level, at least one of administration propriety, dosage, or administration of at least one selected from the group consisting of: Compound (1); Compound (2); a cyclodextrin clathrate of at least one selected from the group consisting of a pharmacologically acceptable salt of Compound (2), Compound (1) and Compound (2); and pantethine trisulfide and a pharmacologically acceptable salt thereof.
[10] The method according to [9], wherein the prediction or determination step comprises estimating the subject's blood urea nitrogen level and/or blood creatinine level based on the blood GDF-15 level, and predicting or determining, based on the blood urea nitrogen level and/or the blood creatinine level, at least one of administration propriety, dosage, or administration of at least one selected from the group consisting of: Compound (1); Compound (2); a cyclodextrin clathrate of at least one selected from the group consisting of a pharmacologically acceptable salt of Compound (2), Compound (1) and Compound (2); and pantethine trisulfide and a pharmacologically acceptable salt thereof.
[11] A method for predicting or determining at least one of administration propriety, dosage, or administration of the above trisulfides to a patient with acute kidney injury induced by an anti-cancer agent, the method comprising: a measurement step of measuring the blood GDF-15 level of the patient with acute kidney injury induced by an anti-cancer agent; and a prediction or determination step of predicting or determining, based on the blood GDF-15 level, administration propriety, dosage, or administration of the above trisulfides to the patient.
[12] A method for producing a prophylactic or therapeutic agent for acute kidney injury induced by an anti-cancer agent, comprising the above trisulfides, the method comprising: a measurement step of measuring the blood GDF-15 level of a subject; and a prediction or determination step of predicting or determining, based on the blood GDF-15 level, an amount of the above trisulfides in the prophylactic or therapeutic agent.
[13] An *in vitro* method for measuring an index for prognosis and/or diagnosis of acute kidney injury induced by an anti-cancer agent, the method comprising: a measurement step of measuring GDF-15 level, as the index, in a sample obtained from a subject.
[14] A method for predicting or determining at least one of administration propriety, dosage, or administration of the above trisulfides to a patient with acute kidney injury induced by an anti-cancer agent, the method comprising: a measurement step of measuring the blood GDF-15 level of the patient.
[15] A method for providing, for the purpose of predicting or determining at least one of administration propriety, dosage, or administration of the above trisulfides to a patient with acute kidney injury induced by an anti-cancer agent, the blood GDF-15 level of the patient, the method comprising: a measurement step of measuring the blood GDF-15 level of the patient.

### Advantageous Effects of Invention

According to the present invention, it is possible to provide a prophylactic or therapeutic agent for acute kidney injury induced by an anti-cancer agent that can exert prophylactic or therapeutic effects at a low dose, with potential for administration by either the oral or parenteral route. In addition, upon preventing or treating acute kidney injury induced by an anti-cancer agent, it is possible to provide a method in which blood growth differentiation factor-15 (GDF-15) level is used as an index for selecting at least one of administration propriety, dosage, or administration of the prophylactic or therapeutic agent.

### Brief Description of Drawings

FIG. 1 shows the relationship between the concentrations of GSSSG and Na₂S₄ and their cell death-suppressing effects, in Example 1.
FIG. 2 shows images of viable cells (middle row) and dead cells (bottom row) when the concentration of GSSSG and Na₂S₄ was each 0.1 µM, in Example 1.

### Description of Embodiments

The present invention is described in detail below; however, the present invention is not limited to the following embodiments.

The prophylactic or therapeutic agent, the prophylactic or therapeutic method, and the other methods described in the present description may be administered or applied to humans.

One embodiment of the present invention relates to a prophylactic or therapeutic agent for acute kidney injury induced by an anti-cancer agent, comprising the above trisulfides (that is, at least one selected from the group consisting of glutathione trisulfide (GSSSG) and a pharmacologically acceptable salt thereof; Compound (1); Compound (2); a cyclodextrin clathrate of at least one selected from the group consisting of a pharmacologically acceptable salt of Compound (2), Compound (1), and Compound (2); and pantethine trisulfide and a pharmacologically acceptable salt thereof).

The above trisulfides may be at least one selected from the group consisting of: Compound (1); Compound (2); a cyclodextrin clathrate of at least one selected from the group consisting of a pharmacologically acceptable salt of Compound (2), Compound (1), and Compound (2); and pantethine trisulfide and a pharmacologically acceptable salt thereof. They may also be at least one selected from the group consisting of Compound (1); Compound (2); and pantethine trisulfide and a pharmacologically acceptable salt thereof.

When R¹ or R² is a C₁₋₆ alkyl group optionally having one or more substituents selected from the group consisting of a carboxy group and -OR⁵; a C₂₋₆ alkyl group having one or more substituents selected from the group consisting of -NR⁶R⁷ and -N⁺R⁹R¹⁰R¹¹; or - (CH₂CH₂O)ₙR⁸ in Formula (1), the hydrophilicity of Compound (1) may be increased, which is expected to improve drug absorption, e.g., in oral ingestion.

R¹ and R² in Formula (1) may each be a hydrogen atom, or an alkyl group such as a methyl group, an ethyl group, a propyl group, a butyl group, a pentyl group, or a hexyl group. These alkyl groups optionally have one or more substituents selected from the group consisting of a carboxy group and -OR⁵, where -OR⁵ includes a hydroxy group, a methoxy group, an ethoxy group, a propoxy group, an isopropoxy group, etc. R¹ and R² in Formula (1) may be an alkyl group such as an ethyl group, a propyl group, a butyl group, a pentyl group, or a hexyl group. These alkyl groups optionally have one or more substituents selected from the group consisting of -NR⁶R⁷, including amino, dimethylamino, etc., and -N⁺R⁹R¹⁰R¹¹, including -N⁺H₃, - N⁺(CH₃)₃, -N⁺(C₂H₆)₃, etc. For example, R¹ and R² optionally each have one or both of an amino group or a carboxy group. R¹ and R² may, for example, be a substituent represented by Formula (10) or (11) below (where "*" denotes a bond). R¹ and R² may also be a substituent represented by -(CH₂CH₂O)ₙR⁸, such as bis(2-ethoxyethyl) ether. Specific examples of compounds represented by Formula (1) include the compound in which both R¹ and R² are hydrogen atoms, the compound in which R¹ is a hydrogen atom and R² is a substituent represented by Formula (10), and the compound in which R¹ is a hydrogen atom and R² is a substituent represented by Formula (11).

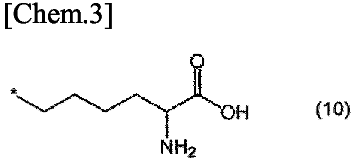

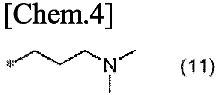

Compounds in the present specification may exist as optical isomers and racemates. The present invention is not limited to any particular optical form; it may be a racemate, one of the enantiomers, or a mixture of enantiomers in any arbitrary ratio.

In Formula (2), R³ may be a hydrogen atom or an alkyl group such as a methyl group, an ethyl group, a propyl group, a butyl group, a pentyl group, or a hexyl group. These alkyl groups optionally have a substituent represented by -OR⁵. Examples of -OR⁵ include a hydroxy group, a methoxy group, an ethoxy group, a propoxy group, an isopropoxy group, etc. R³ in Formula (2) may be an alkyl group such as an ethyl group, a propyl group, a butyl group, a pentyl group, or a hexyl group. These alkyl groups optionally have one or more substituents selected from the group consisting of -NR⁶R⁷ and - N⁺R⁹R¹⁰R¹¹. -NR⁶R⁷ includes amino, dimethylamino, etc., and - N⁺R⁹R¹⁰R¹¹ includes -N⁺H₃, -N⁺(CH₃)₃, -N⁺(C₂H₆)₃, etc. R³ in Formula (2) may also be a group represented by -(CH₂CH₂O)ₙR⁸, and - (CH₂CH₂O)ₙR⁸ may be bis(2-ethoxyethyl) ether. For example, R³ in Formula (2) may be a substituent represented by Formula (30) or (31) below (where "*" denotes a bond).

Specific examples of compounds represented by Formula (2) include the compound in which R³ is a substituent represented by Formula (30), and the compound in which R³ is a substituent represented by Formula (31).

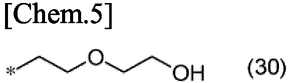

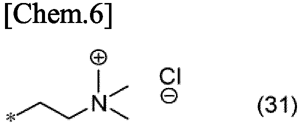

A compound represented by Formula (2) generally has high hydrophilicity, which is expected to improve drug absorption, e.g., in oral ingestion.

A compound represented by Formula (2), a pharmacologically acceptable salt thereof, and Compound (1) can be produced, for example, by the method described in Patent Literature 4.

A cyclodextrin clathrate of at least one selected from the group consisting of a pharmacologically acceptable salt of Compound (2), Compound (1), and Compound (2) is formed by inclusion of at least one selected from the group consisting of a pharmacologically acceptable salt of Compound (2), Compound (1), and Compound (2) by cyclodextrin or a derivative thereof.

The cyclodextrin used may be α-cyclodextrin, β-cyclodextrin, γ-cyclodextrin, or a derivative thereof. A derivative of cyclodextrin herein refers to a compound in which at least one hydrogen atom of a hydroxyl group of the cyclodextrin is substituted by an alkyl group optionally having a substituent, or by a sugar. Examples of cyclodextrin derivatives include methyl-α-cyclodextrin, methyl-β-cyclodextrin, methyl-γ-cyclodextrin, dimethyl-α-cyclodextrin, dimethyl-β-cyclodextrin, dimethyl-γ-cyclodextrin, hydroxyethyl-α-cyclodextrin, hydroxyethyl-β-cyclodextrin, hydroxyethyl-γ-cyclodextrin, 2-hydroxypropyl-α-cyclodextrin, 2-hydroxypropyl-β-cyclodextrin, 2-hydroxypropyl-γ-cyclodextrin, glucosyl-α-cyclodextrin, glucosyl-β-cyclodextrin, glucosyl-γ-cyclodextrin, maltosyl-α-cyclodextrin, maltosyl-β-cyclodextrin, maltosyl-γ-cyclodextrin, sulfobutylether-α-cyclodextrin, sulfobutylether-β-cyclodextrin, and sulfobutylether-γ-cyclodextrin.

The cyclodextrin clathrate of at least one selected from the group consisting of a pharmacologically acceptable salt of Compound (2), Compound (1), and Compound (2) can be produced, for example, by the method described in Patent Literature 4.

Pantethine trisulfide is a compound represented by Formula (3) below.

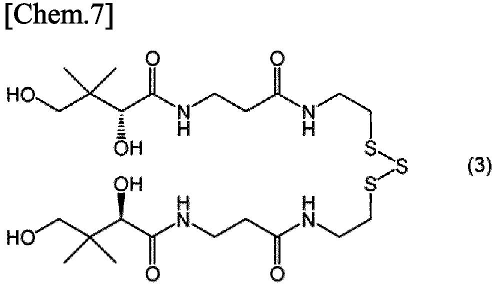

Pantethine trisulfide and a pharmacologically acceptable salt thereof can be produced, for example, by the method described in Patent Literature 3.

In one embodiment of the present invention, examples of a pharmacologically acceptable salt include salts with an inorganic acid such as hydrochloric acid, hydrobromic acid, sulfuric acid, nitric acid, or phosphoric acid; salts with an organic acid such as acetic acid, succinic acid, fumaric acid, maleic acid, tartaric acid, citric acid, lactic acid, stearic acid, benzoic acid, methanesulfonic acid, ethanesulfonic acid, or p-toluenesulfonic acid; or salts with an alkali metal such as sodium or potassium; salts with an alkaline-earth metal such as calcium or magnesium; ammonium salts; and salts with an amino acid such as arginine.

In one embodiment of the present invention, the above trisulfides may include crystal polymorphs. The crystalline form is not limited; any crystal form may be used, whether a single polymorph or a mixture of multiple forms. An amorphous form is also encompassed by one embodiment of the present invention, the above trisulfides. An anhydrate or a solvate (particularly a hydrate) is likewise included in one embodiment of the present invention, the above trisulfides.

In one embodiment of the present invention, the prophylactic or therapeutic agent for acute kidney injury induced by an anti-cancer agent can be administered orally in the form of, for example, tablets, capsules, powders, granules, liquids, or syrups. Although the prophylactic or therapeutic agent of the present invention may also be administered parenterally, for example in the form of injections, infusions, or suppositories, administering it orally can reduce the burden on the subject (e.g., a cancer patient) to be administered. The prophylactic or therapeutic agent of the present invention can be formulated into such dosage forms by known formulation techniques. In the case of a solid preparation, a pharmacologically acceptable excipient may be employed in the course of formulation, for example starch, lactose, sucrose, glucose, crystalline cellulose, carboxycellulose, carboxymethylcellulose, carboxyethylcellulose, calcium phosphate, magnesium stearate, Gum Arabic, etc. If necessary, a lubricant, a binder, a disintegrant, a coating agent, a coloring agent, etc. may be employed. In the case of a liquid preparation, a stabilizer, a solubilizing aid, a suspending agent, an emulsifier, a buffering agent, a preservative, etc. may be employed.

In one embodiment of the present invention, the dosage of the prophylactic or therapeutic agent for acute kidney injury induced by an anti-cancer agent is a therapeutically effective amount, which varies depending on the patient's condition, age, route of administration, dosage form, and so on. Typically, for an adult, from 0.5-2000 mg of the compound per day can be administered; preferably 1-800 mg per day administered either in a single dose, or divided into several doses, or administered continuously by drip infusion over successive days. When the dosage of the prophylactic or therapeutic agent of the present invention is within this range, it is considered that prophylactic or therapeutic effects with respect to acute kidney injury induced by an anti-cancer agent will be enhanced.

The acute kidney injury induced by an anti-cancer agent may be an acute kidney injury caused by acute tubular necrosis induced by the anti-cancer agent. Anti-cancer drugs that may cause acute kidney injury due to acute tubular necrosis or acute kidney injury include cytotoxic anti-cancer drugs, molecular target drugs, bisphosphonate preparations, interferon preparations, etc.

Examples of cytotoxic anti-cancer agents that may cause acute kidney injury due to acute tubular necrosis or acute kidney injury include platinum preparations such as cisplatin, carboplatin, and oxaliplatin; alkylating agents such as cyclophosphamide and ifosfamide; antimetabolites such as methotrexate, pemetrexed, gemcitabine, and pentostatin; and antibiotics such as mitomycin C. Examples of molecular target drugs that may cause acute kidney injury due to acute tubular necrosis or acute kidney injury include bevacizumab, imatinib, sorafenib, nivolumab, ipilimumab, sunitinib, cetuximab and the like. Examples of bisphosphonate preparations that may cause acute kidney injury due to acute tubular necrosis or acute kidney injury include zoledronic acid, pamidronic acid and the like. Acute kidney injury induced by an anti-cancer agent or acute kidney injury caused by acute tubular necrosis induced by an anti-cancer agent includes not only acute kidney injury that arises from administration of any of these anti-cancer agents singly, but also acute kidney injury that arises from the combination of two or more of these anti-cancer agents.

Another embodiment of the present invention is a method for predicting or determining at least one of administration propriety, dosage, or administration of the above trisulfides to a subject, the method comprising: a measurement step of measuring the subject's blood GDF-15 level, and a prediction or determination step of predicting or determining, based on the blood GDF-15 level, at least one of administration propriety, dosage, or administration.

The above prediction or determination step may include estimating the subject's blood urea nitrogen level and/or blood creatinine level based on the blood GDF-15 level, and predicting or determining, based on the blood urea nitrogen level and/or blood creatinine level, at least one of administration propriety, dosage, or administration of the above trisulfides.

A further embodiment of the present invention is a method for producing a prophylactic or therapeutic agent for acute kidney injury induced by an anti-cancer agent, comprising the above trisulfides, the method comprising: a measurement step of measuring the blood GDF-15 level of a subject, and a prediction or determination step of predicting or determining, based on the blood GDF-15 level, an amount of the above trisulfides in the prophylactic or therapeutic agent.

In the above method of producing the prophylactic or therapeutic agent, the act of predicting or determining the amount of the above trisulfides in the prophylactic or therapeutic agent may be performed by the subject who will receive the prophylactic or therapeutic agent. The prophylactic or therapeutic agent is not limited to a single formulation but may be a combination of multiple preparations. Where it is a single formulation, the subject may choose a preparation that contains an appropriate quantity of the above trisulfides, on the basis of the measured GDF-15 value. Where it is a combination of multiple formulations, the subject's choice of which formulations to combine would enable them to control the amount of the above trisulfides in the prophylactic or therapeutic agent.

A still further embodiment of the present invention is an *in vitro* method for measuring an index for the prognosis and/or diagnosis of acute kidney injury induced by an anti-cancer agent, the method comprising: a measurement step of measuring the GDF-15 level in a sample obtained from a subject, as the above index. This method may further include estimating the blood urea nitrogen level and/or blood creatinine level of the subject based on the blood GDF-15 level, and then performing a prediction or determination step of predicting or determining, based on the blood urea nitrogen level and/or blood creatinine level, at least one of administration propriety, dosage, or administration of the above trisulfides to the subject.

Another embodiment of the present invention is a method for predicting or determining at least one of administration propriety, dosage, or administration of the above trisulfides to a patient with acute kidney injury induced by an anti-cancer agent, the method comprising: a measurement step of measuring the blood GDF-15 level of the patient with acute kidney injury induced by an anti-cancer agent. This method may further include estimating the patient's blood urea nitrogen level and/or blood creatinine level based on the blood GDF-15 level, and a prediction or determination step of predicting or determining, based on the blood urea nitrogen level and/or blood creatinine level, at least one of administration propriety, dosage, or administration of the above trisulfides.

A still further embodiment of the present invention is a method for providing, for the purpose of predicting or determining at least one of administration propriety, dosage, or administration of the above trisulfides to a patient with acute kidney injury induced by an anti-cancer agent, the patient's blood GDF-15 level, the method comprising: a measurement step of measuring the blood GDF-15 level of the patient with acute kidney injury induced by an anti-cancer agent. This method may further include estimating the patient's blood urea nitrogen level and/or blood creatinine level based on the blood GDF-15 level, and then predicting or determining, based on the blood urea nitrogen level and/or blood creatinine level, at least one of administration propriety, dosage, or administration.

In the present specification, the "subject" may be a patient who has been diagnosed with cancer and is scheduled to receive an anti-cancer agent, a patient who is already undergoing treatment with an anti-cancer agent, or a patient who has developed acute kidney injury induced by an anti-cancer agent (particularly a patient who has developed acute kidney injury due to acute tubular necrosis caused by administration of the anti-cancer agent).

The blood GDF-15 level that serves as a criterion for predicting or determining at least one of administration propriety, dosage, or administration of the above trisulfides will vary depending on symptoms, age, administration route, dosage form, etc., but for a typical adult, 0.05 ng/mL-100 ng/mL, preferably 0.1 ng/mL-10 ng/mL, is mentioned. If the blood GDF-15 level is within these ranges, one can judge that administering the above trisulfides would be desirable, in which case the methods and/or doses described herein can be applied.

### Examples

The present invention will be described in further detail with reference to the following experimental examples; however, the present invention is not limited thereto.

### Experimental Example 1: Evaluation (in vitro) of the Suppression Effect on Cisplatin-Induced Cytotoxicity in Renal Tubular Cells

Human proximal renal tubular cells (HK-2) were seeded into a 96-well plate containing medium at a density of 5×10³ cells/well. After confirming that the seeded cells had adhered to the plate, each test compound (GSSSG or Na₂S₄) was added so that the final concentration was 0 µM, 0.01 µM, 0.02 µM, 0.10 µM, 0.39 µM, 1.56 µM, 6.25 µM, 25 µM, or 100 µM, followed by standing at 37°C for 0.5 hours. Each test compound was dissolved in PBS and then added. A final concentration of 0 µM indicates that only PBS was added to the well.

The supernatant in each well was removed, and the cells were washed with PBS. Fresh medium was then added to each well, cisplatin was added at a final concentration of 20 µM, and the plate was left standing at 37°C for 24 hours. Thereafter, cell viability was evaluated using cell counting kit-8 (Dojindo).

The composition of the medium used in Example 1 was as follows: D-MEM/Ham's F-12 (FUJIFILM Wako Pure Chemical Corporation) + Penicillin-Streptomycin-Amphotericin B (FUJIFILM Wako Pure Chemical Corporation) + GlutaMAX (Thermo Fisher Scientific) + 10% FBS.

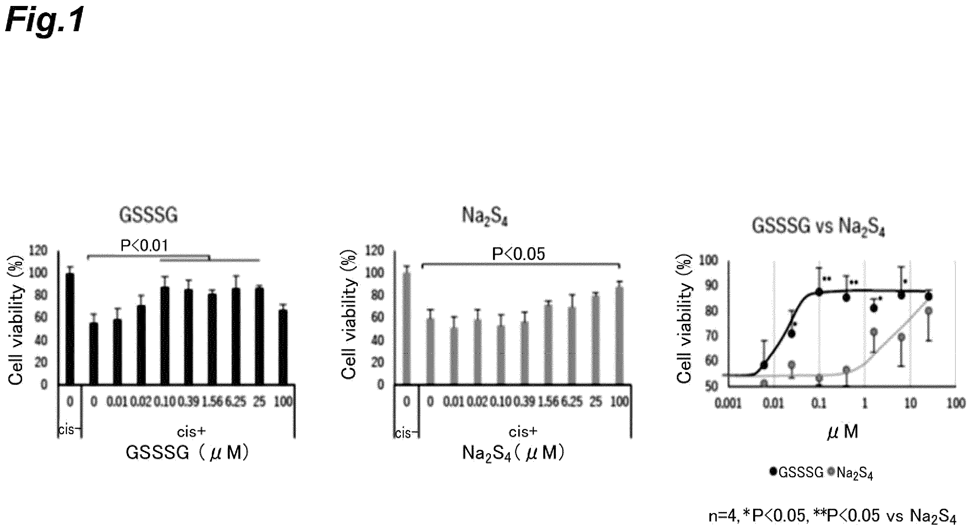

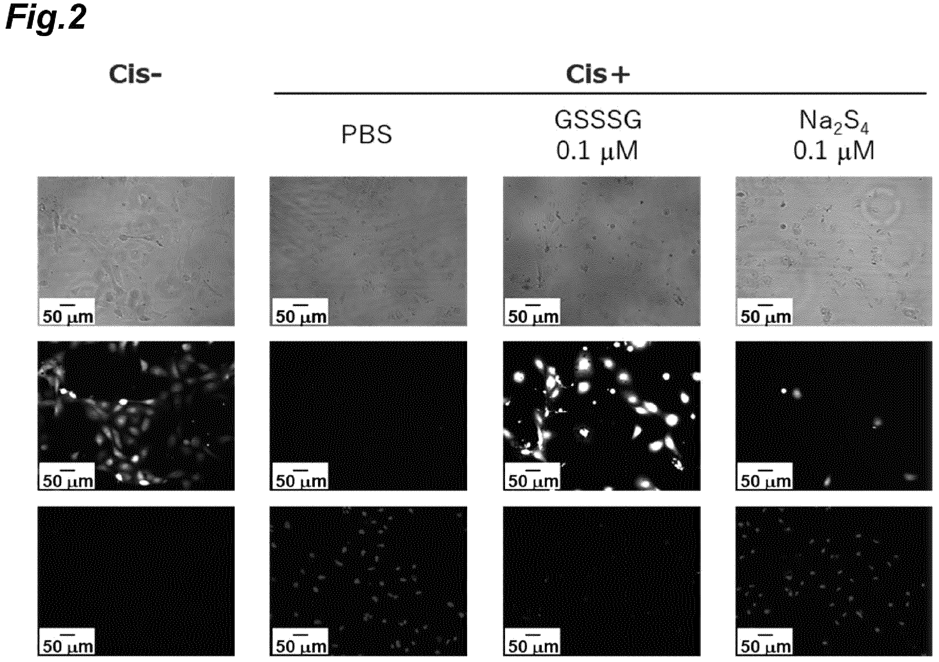

FIG. 1 shows the cell viability at each test compound concentration in Example 1. FIG. 2 shows transmission images (top row) and fluorescence images of viable cells (middle row) and dead cells (bottom row) for the case in which the test compound concentration was 0.1 µM. In FIGS. 1 and 2, "Cis-" indicates that cisplatin was not added, and "Cis+" indicates that cisplatin was added.

As shown in FIG. 1, both GSSSG (an organic trisulfide) and Na₂S₄ (an inorganic polysulfide) showed a tendency to suppress cell death in a concentration-dependent manner. However, GSSSG exhibited a significant cell death-suppressing effect even at a low concentration of 0.1 µM, whereas Na₂S₄ only showed a significant cell death-suppressing effect at 100 µM. This suggests that the cell death-suppressing effect of GSSSG may be about 1000 times stronger. In addition, as shown in FIG. 2, it was clear that, even when cisplatin was added, the number of viable renal tubular cells was notably higher in the group to which GSSSG was administered.

## Claims

1. A prophylactic or therapeutic agent for acute kidney injury induced by an anti-cancer agent, comprising at least one selected from the group consisting of:
glutathione trisulfide and a pharmacologically acceptable salt thereof;
a compound represented by Formula (1): 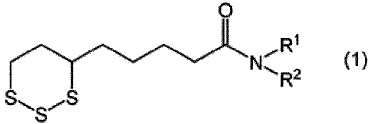
wherein R¹ and R² are each independently a hydrogen atom; a C₁₋₆ alkyl group optionally having one or more substituents selected from the group consisting of a carboxy group and -OR⁵; a C₂₋₆ alkyl group having one or more substituents selected from the group consisting of - NR⁶R⁷ and -N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5;
a compound represented by Formula (2): 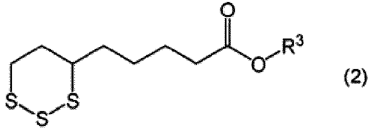
wherein R³ is a hydrogen atom; a C₁₋₆ alkyl group optionally having one or more substituents selected from the group consisting of a carboxy group and -OR⁵; a C₂₋₆ alkyl group having one or more substituents selected from the group consisting of -NR⁶R⁷ and - N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5;
a cyclodextrin clathrate of at least one selected from the group consisting of a pharmacologically acceptable salt of the compound represented by Formula (2), the compound represented by Formula (1) and the compound represented by Formula (2); and
pantethine trisulfide and a pharmacologically acceptable salt thereof.

2. The prophylactic or therapeutic agent according to claim 1, wherein the prophylactic or therapeutic agent comprises at least one selected from the group consisting of:
glutathione trisulfide and a pharmaceutically acceptable salt thereof;
the compound represented by Formula (1);
the compound represented by Formula (2); and
pantethine trisulfide and a pharmaceutically acceptable salt thereof.

3. The prophylactic or therapeutic agent according to claim 1, wherein the acute kidney injury induced by the anti-cancer agent is acute kidney injury due to acute tubular necrosis induced by the anti-cancer agent.

4. The prophylactic or therapeutic agent according to claim 1, wherein the anti-cancer agent is at least one selected from the group consisting of cisplatin, carboplatin, oxaliplatin, cyclophosphamide, ifosfamide, methotrexate, pemetrexed, gemcitabine, pentostatin, and mitomycin C.

5. The prophylactic or therapeutic agent according to any one of claims 1 to 4, wherein the anti-cancer agent is at least one selected from the group consisting of cisplatin, carboplatin, and oxaliplatin.

6. A method for determining, for a patient with acute kidney injury induced by an anti-cancer agent, at least one of administration propriety, dosage, or administration of at least one selected from the group consisting of
glutathione trisulfide and a pharmacologically acceptable salt thereof;
a compound represented by Formula (1): 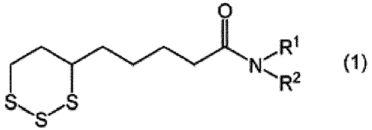
wherein R¹ and R² are each independently a hydrogen atom; a C₁₋₆ alkyl group optionally having one or more substituents selected from the group consisting of a carboxy group and -OR⁵; a C₂₋₆ alkyl group having one or more substituents selected from the group consisting of - NR⁶R⁷ and -N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5;
a compound represented by Formula (2): 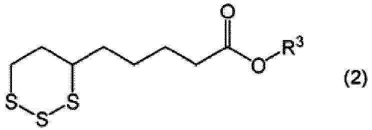
wherein R³ is a hydrogen atom; a C₁₋₆ alkyl group optionally having one or more substituents selected from the group consisting of a carboxy group and -OR⁵; a C₂₋₆ alkyl group having one or more substituents selected from the group consisting of -NR⁶R⁷ and - N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5;
a cyclodextrin clathrate of at least one selected from the group consisting of a pharmacologically acceptable salt of the compound represented by Formula (2), the compound represented by Formula (1) and the compound represented by Formula (2); and
pantethine trisulfide and a pharmacologically acceptable salt thereof,
the method comprising:
a measurement step of measuring a blood GDF-15 level of a patient with acute kidney injury induced by an anti-cancer agent; and
a determination step of determining, based on the blood GDF-15 level, at least one of administration propriety, dosage, or administration of at least one selected from the group consisting of glutathione trisulfide and a pharmacologically acceptable salt thereof; the compound represented by Formula (1); the compound represented by Formula (2); the cyclodextrin clathrate of at least one selected from the group consisting of the pharmacologically acceptable salt of the compound represented by Formula (2), the compound represented by Formula (1) and the compound represented by Formula (2); and pantethine trisulfide and a pharmacologically acceptable salt thereof.

7. A method for producing a prophylactic or therapeutic agent for acute kidney injury induced by an anti-cancer agent, comprising at least one selected from the group consisting of:
glutathione trisulfide and a pharmacologically acceptable salt thereof;
a compound represented by Formula (1): 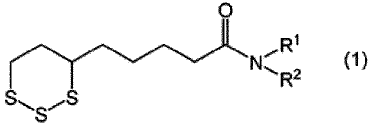
wherein R¹ and R² are each independently a hydrogen atom; a C₁₋₆ alkyl group optionally having one or more substituents selected from the group consisting of a carboxy group and -OR⁵; a C₂₋₆ alkyl group having one or more substituents selected from the group consisting of - NR⁶R⁷ and -N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5;
a compound represented by Formula (2): 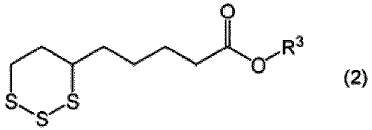
wherein R³ is a hydrogen atom; a C₁₋₆ alkyl group optionally having one or more substituents selected from the group consisting of a carboxy group and -OR⁵; a C₂₋₆ alkyl group having one or more substituents selected from the group consisting of -NR⁶R⁷ and - N⁺R⁹R¹⁰R¹¹; or -(CH₂CH₂O)ₙR⁸, wherein R⁵, R⁶, R⁷, R⁸, R⁹, R¹⁰, and R¹¹ are each independently a hydrogen atom or a C₁₋₃ alkyl group, and n is an integer of 2 to 5;
a cyclodextrin clathrate of at least one selected from the group consisting of a pharmacologically acceptable salt of the compound represented by Formula (2), the compound represented by Formula (1) and the compound represented by Formula (2); and
pantethine trisulfide and a pharmacologically acceptable salt thereof,
the method comprising:
a measurement step of measuring a blood GDF-15 level of a subject; and
a determination step of determining, based on the blood GDF-15 level, an amount of at least one selected from the group consisting of glutathione trisulfide and a pharmacologically acceptable salt thereof; the compound represented by Formula (1); the compound represented by Formula (2); the cyclodextrin clathrate of at least one selected from the group consisting of the pharmacologically acceptable salt of the compound represented by Formula (2), the compound represented by Formula (1) and the compound represented by Formula (2); and pantethine trisulfide and a pharmacologically acceptable salt thereof, in the prophylactic or therapeutic agent.

8. An *in vitro* method for measuring an index for prognosis and/or diagnosis of acute kidney injury induced by an anti-cancer agent,
the method comprising: a measurement step of measuring a GDF-15 level, as the index, in a sample obtained from a subject.
